# EUROPEAN PATENT APPLICATION

(11) **EP 3 466 317 A1**
(43) Date of publication of application: **10.04.2019**
(21) Application number: 17802681.1
(22) Date of filing: 18.05.2017
(51) Int. Cl.: A61B 1/04, A61B 1/00, A61B 1/05, A61B 1/06, G02B 23/24

(54) **ENDOSCOPIC APPARATUS**

(30) Priority: 24.05.2016 JP 2016103674
(71) Applicant: Kairos Co., Ltd., Tokyo 1050014 (JP)
(72) Inventor: CHIBA, Toshio, Tokyo 101-0062 (JP); TANIOKA, Kenkichi, Tokyo 162-0814 (JP); YAMASHITA, Hiromasa, Tokyo 101-0062 (JP)
(74) Representative: Müller-Boré & Partner Patentanwälte PartG mbB
(86) International application number: PCT/JP2017/018744
(87) International publication number: WO 2017/204089

(57) **Abstract**

A high-resolution and compact endoscopic apparatus is provided. The endoscopic apparatus comprises: an insertion unit (110) configured to be inserted into a body cavity and guide light from an object (A); an illumination device (120) attached to the insertion unit (110) and illuminating the object (A); and an imaging device (130) comprising 8K-level or higher-level pixels arranged in a matrix form. The imaging device (130) receives light reflected from the object (A) and guided through the insertion unit and outputs image data of the object (A). The pitch of the pixels of the imaging device (130) is equal to or larger than the longest wavelength of illumination light emitted from the illumination means.

## Description

### [Technical Field]

The present invention relates to an endoscopic apparatus.

### [Background Art]

Endoscopic apparatuses are widely used which are configured to insert an elongate insertion unit into a body cavity and capture images inside the body cavity (see Patent Document 1, for example). On the other hand, a high-resolution video technique called 8K or the like is put into practical use. Accordingly, it is proposed to apply such a high-resolution video technique to the endoscopic apparatuses (see Patent Document 2, for example).

### [Prior Art Documents]

### [Patent Documents]

[Patent Document 1] JP6-277173A
[Patent Document 2] JP2015-077400A

### [Summary of the Invention]

### [Problems to be solved by the Invention]

True resolution (image denseness) of 8K cannot necessarily be achieved on a display by simply setting the number of pixels of an image sensor to 8K (7680x4320 pixels).

To truly realize a resolution of 8K, it is required that "the size of pixels be large." If the size of pixels of an image sensor is unduly small, the captured images cannot be resolved due to the diffraction limit of light, resulting in blurred images. When applied to an endoscopic apparatus, a large-sized image sensor may be difficult to use without any modification because the diameter of a built-in lens of the endoscopic apparatus is very small due to the limitation that the endoscopic apparatus has to be inserted into a body cavity.

It is conceivable to enlarge the diameter of a light beam guided in the endoscopic apparatus to the entire area of the image sensor using a magnifying lens. However, the higher the magnification (the farther the focal point distance), the larger the area of an image circle on the screen increases, but the range of an operative field in which the reflected light can be obtained narrows. This reduces the amount of light (photons) received by the image sensor so that the image becomes dark. Thus, the size and brightness of the image circle are in a trade-off relationship and they are difficult to achieve at the same time.

Moreover, cameras for 8K are very large and it is difficult to attach such a large camera to an endoscopic apparatus. Furthermore, endoscopic apparatuses to which cameras for 8K are attached are large and thus difficult to handle.

The present invention has been made in view of the above circumstances and an object of the present invention is to provide a high-resolution and compact endoscopic apparatus.

### [Means for solving the Problems]

To achieve the above object, the endoscopic apparatus according to the present invention comprises an insertion unit configured to be inserted into a body cavity and guide light from an object, an illumination means attached to the insertion unit and illuminating the object, and an imaging element comprising 8K-level or higher-level pixels arranged in a matrix form. The imaging element receives light reflected from the object and guided through the insertion unit and outputs imaging signals of the object The pixels of the imaging element have a pitch equal to or larger than the longest wavelength of illumination light emitted from the illumination means.

The illumination means comprises, for example, an LED element and a light guiding means that guides light output from the LED element. When the illumination light output from the illumination means includes light having a plurality of frequencies within a sensitive frequency band of the imaging element, the pitch of the pixels is preferably larger than a value corresponding to the highest frequency among the frequencies of light having intensity equal to or higher than a predetermined threshold.

The imaging element may be provided with a means that converts a pixel voltage to pixel data, and the endoscopic apparatus may further comprise an image processing unit that creates frame data from the pixel data provided from the imaging element and processes the frame data, a display device that is connected to the image processing unit and displays the frame data, and a cable of 1 to 10 m that connects between the imaging element and the image processing unit.

### [Effect of the Invention]

According to the present invention, a truly high-resolution image can be obtained with limited influence of the diffraction of illumination light.

### [Brief Description of Drawings]

FIG. 1 is a diagram illustrating the configuration of an endoscopic apparatus according to an embodiment of the present invention.
FIG. 2 is a diagram illustrating a detailed configuration of the endoscopic apparatus illustrated in FIG. 1.
FIG. 3 is a diagram for describing the pixel pitch of an imaging element illustrated in FIG. 2.
FIG. 4 is a block diagram illustrating the detailed configuration of a control device illustrated in FIG. 1.
FIG. 5 is a set of diagrams for describing the aperture ratio of cylindrical parts of endoscopic apparatuses, wherein (a) illustrates the configuration of an embodiment of the present invention and (b) illustrates a conventional configuration.
FIG. 6 is a diagram illustrating the configuration of an insertion unit according to a first modified example.
FIG. 7 is a diagram illustrating the configuration of an insertion unit according to a second modified example.
FIG. 8 is a diagram illustrating the configuration of an insertion unit according to a third modified example.
FIG. 9 is a diagram illustrating the configuration of an insertion unit according to a fourth modified example.

### [Mode(s) for Carrying out the Invention]

The endoscopic apparatus according to an embodiment of the present invention will be described in detail with reference to the drawings.

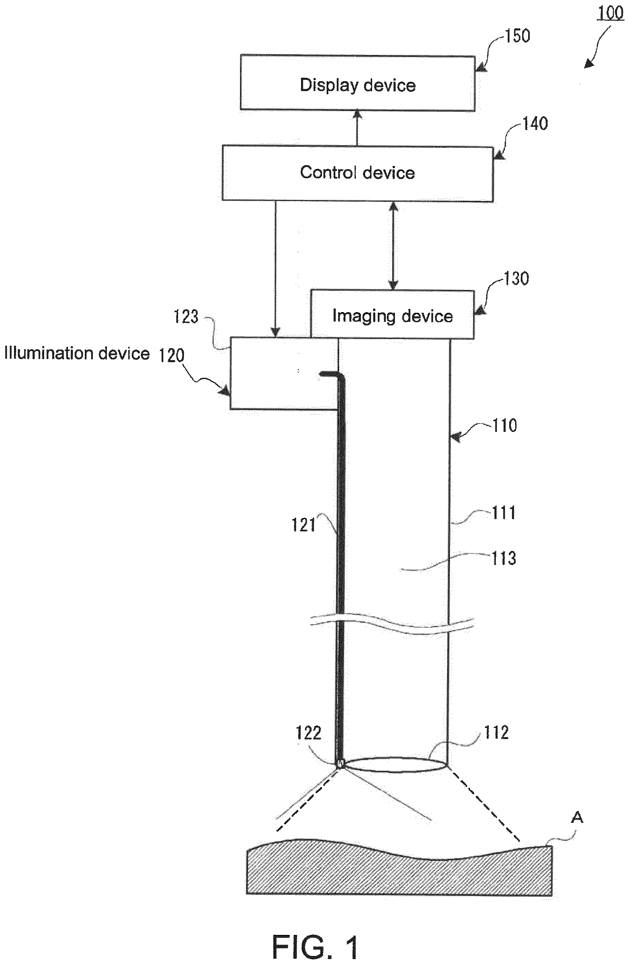

The endoscopic apparatus 100 according to the present embodiment is a rigid scope that is primarily used as a laparoscope or a luminal scope. As illustrated in FIG. 1, the endoscopic apparatus 100 comprises an insertion unit 110, an illumination device 120, an imaging device 130, a control device 140, and a display device 150.

The insertion unit 110 is an elongate member configured to be inserted into a body cavity of a person under test or the like. The insertion unit 110 comprises a tubular (cylindrical) part 111, an objective lens 112, and a hollow light guide region 113.

The cylindrical part 111 is a member configured such that a metal material such as a stainless steel material, a hard resin material, or the like is formed into a cylindrical or elliptical cylindrical shape having, for example, a diameter of 8 mm to 9 mm. The illumination device 120 is detachably attached to a side surface in the vicinity of the base end of the cylindrical part 111 and the imaging device 130 is detachably attached to the base end portion of the cylindrical part 111.

The objective lens 112 is a light guide means that introduces light emitted from the illumination device 120 and reflected by an object A in the body cavity. The objective lens 112 is composed, for example, of a wide-angle lens. The objective lens 112 is disposed so as to be exposed from the distal end surface of the insertion unit 110. The objective lens 112 converges the reflected light from the object A and forms an image of the object A on the imaging surface of the imaging device 130 via the hollow light guide region 113. The side surface of the objective lens 112 is fixed to the inner wall surface of the distal end portion of the cylindrical part 111 using an adhesive or the like, and the distal end surface of the insertion unit 110 is thus sealed.

The hollow light guide region 113 is a space arranged between the base end portion and distal end portion of the cylindrical part 111 and serves as a light guide means that guides the light having passed through the objective lens 112 to the imaging device 130.

The illumination device 120 comprises an optical fiber 121, a diffusion layer 122, and a light source unit 123. The optical fiber 121 is led out from the light source unit 123 and fixed to the inner surface of the cylindrical part 111 with an adhesive or the like and extends to the diffusion layer 122 at the distal end portion of the cylindrical part 111.

The diffusion layer 122 diffuses and outputs the light which is supplied from the light source unit 123 via the optical fiber 121. The diffusion layer 122 is composed, for example, of a diffusing plate and/or a diffusing lens that diffuse the incident light and outputs the diffused light.

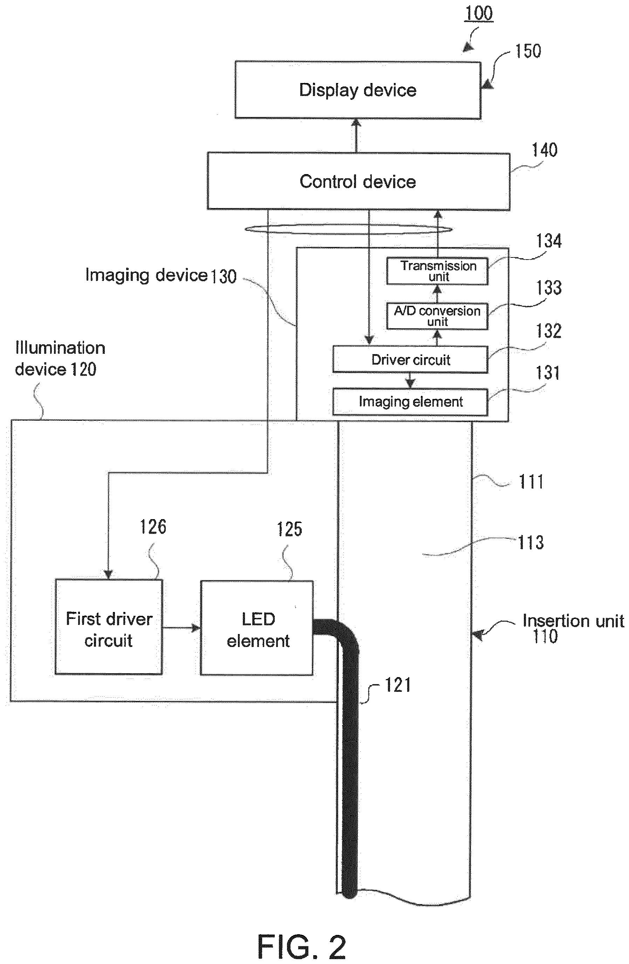

The light source unit 123 supplies light for illuminating the object A to the base end portion of the optical fiber 121. As illustrated in FIG. 2, the light source unit 123 comprises a light emitting diode (LED) element 125 and a driver circuit 126.

The LED element 125 incorporates elements that emit light of three colors of red (R), green (G), and blue (B) and irradiates the incident end of the optical fiber 121 with white light obtained by color mixing.

The driver circuit 126 drives the LED element 125 under the control by the control device 140. The driver circuit 126 has a function of dimming control of the LED element 125 by PWM control or the like under the control by the control device 140.

The imaging device 130, which is detachably attached to the base end portion of the insertion unit 110, captures an image of the object A with the incident light having passed through the hollow part 113 of the cylindrical part 111 and supplies the captured image to the control device 140. More specifically, as illustrated in FIG. 2, the imaging device 130 is composed of an imaging element 131, a driver circuit 132, an AID conversion unit 133, and a transmission unit 134.

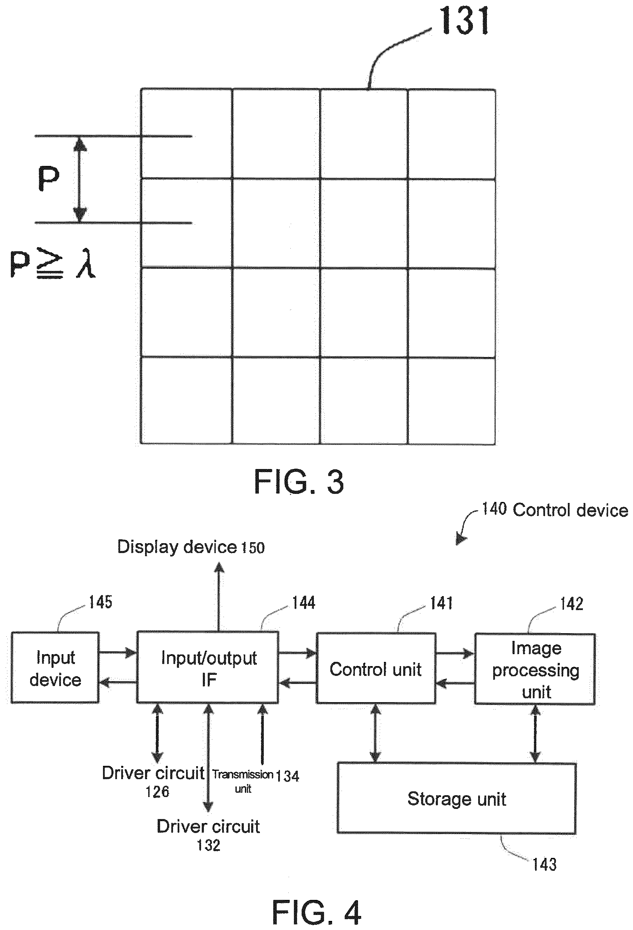

The imaging element 131 is composed of a so-called 8K color image sensor, that is, a color image sensor of 7680×4320 pixels. As illustrated in FIG. 3, the pitch P of pixels of the imaging element 131 has a size equal to or larger than the diffraction limit of primary light used for illumination of the object A. Specifically, the pitch P is set to a value larger than a reference wavelength λ corresponding to the wavelength of the illumination light emitted from the diffusion layer 122, that is, the wavelength of the emission light of the LED element 125. When the illumination light includes light having a plurality of wavelengths, the reference wavelength λ means the wavelength of light having the longest wavelength among the three primary colors of light which constitute the illumination light, that is, the wavelength of the primary component of red light. That is, the reference wavelength λ means the wavelength with the largest energy in the spectral region corresponding to red. The imaging element 131 may comprise pixels equivalent to or larger than 8K.

The driver circuit 132 controls the start and end of exposure of the imaging element 131 under the control by the control device 140 and reads out the voltage signal of each pixel (pixel voltage). The A/D conversion unit 133 converts the pixel voltage read out from the imaging element 131 by the driver circuit 132 into digital data (image data) and outputs the digital data to the transmission unit 134. The transmission unit 134 outputs the luminance data, which is output from the A/D conversion unit 133, to the control device 140.

The control device 140 controls the endoscopic apparatus 100 as a whole. As illustrated in FIG. 4, the control device 140 comprises a control unit 141, an image processing unit 142, a storage unit 143, an input/output interface (IF) 144, and an input device 145.

The control unit 141, which is composed of a central processing unit (CPU), memories, and other necessary components, controls the storage unit 143 to store the luminance data transmitted from the transmission unit 134, controls the image processing unit 142 to process the image data, and controls the display device 150 to display the processed image data. The control unit 141 further controls the driver circuits 126 and 132.

The image processing unit 142, which is composed of an image processor and other necessary components, processes the image data stored in the storage unit 143 under the control by the control unit 141 and reproduces and accumulates the image data of each frame (frame data). The image processing unit 142 also performs various image processes on the image data of each frame unit stored in the storage unit 143. For example, the image processing unit 142 performs a scaling process for enlarging/reducing each image frame at an arbitrary magnification.

The storage unit 143 stores the operation program for the control unit 141, the operation program for the image processing unit 142, the image data received from the transmission unit 134, the frame data reproduced and processed by the image processing unit 142, etc.

The input/output IF 144 controls transmission and reception of data between the control unit 141 and an external device. The input device 145, which is composed of a keyboard, a mouse, buttons, a touch panel, and other necessary components, supplies an instruction from the user to the control unit 141 via the input/output IF 144.

The display device 150, which is composed of a liquid crystal display device or the like having a display pixel number corresponding to 8K, displays an operation screen, a captured image, a processed image, etc. under the control by the control device 140.

Unlike cameras for television broadcasting, the endoscopic apparatus is used in a dedicated facility. The imaging device 130 attached to the insertion unit 110 is therefore connected to the control device 140 via cables of about several meters. The control device 140 and the display device 150 are placed on a table or the like.

The illumination device 120 and the imaging device 130 are separated from the control device 140. Accordingly, the structures attached to the insertion unit 110 are reduced in weight and size, and handling of the insertion unit 110 is thus relatively easy. Moreover, the cables connecting the illumination device 120 and the imaging device 130 to the control device 140 are at most 1 to 10 m in an operation room, and such cables are different from those for a broadcasting site, which may exceed several 100 m in some cases. Thus, signal deterioration due to the cables is small and there is almost no adverse effect by separation.

The operation of the endoscopic apparatus 100 having the above configuration will then be described. When using the endoscopic apparatus 100, the user (practitioner) operates the input device 145 to input an instruction to turn on the endoscopic apparatus 100. In response to this instruction, the control unit 141 turns on the driver circuits 126 and 132.

The driver circuit 126 turns on the LED element 125 while the driver circuit 132 starts imaging with the imaging element 131. The white light output from the LED element 125 is guided through the optical fiber 121 and diffused by the diffusion layer 122 for irradiation.

The imaging element 131 captures a video footage through the objective lens 112 and the hollow light guide part 113. The pitch P of pixels of the imaging element 131 is equal to or larger than the wavelength A of the primary light in the maximum wavelength region of the primary light of the illumination light. The pitch P of pixels is therefore larger than almost all the wavelengths of the illumination light. Thus, the imaging element 131 can acquire high-quality images with limited influence of the light diffraction.

The driver circuit 132 sequentially reads out the pixel voltages of respective pixels from the imaging element 131, and the read out pixel voltages are converted by the A/D conversion unit 133 into digital image data, which are sequentially transmitted from the transmission unit 134 to the control device 140 via the cables.

The control unit 141 of the control device 140 sequentially receives the transmitted image data via the input/output IF 144 and in turn stores the image data in the storage unit 143.

Under the control by the control unit 141, the image processing unit 142 processes the image data stored in the storage unit 143 to reproduce the frame data and may perform additional processing thereon as appropriate.

The control unit 141 appropriately reads out the frame data stored in the storage unit 143 and supplies the frame data to the display device 150 via the input/output IF 144 for display.

The user inserts the insertion unit 110 into the body cavity while confirming the display on the display device 150. When the insertion unit 110 is inserted in the body cavity, the object A is illuminated with light from the diffusion layer 122 and the imaging device 131 captures an image of the object A, which is displayed on the display device 150.

Here, the field of view of the endoscopic apparatus 100 has limitations because the inner diameter of the cylindrical part 111 is small. For observation of a relatively wide range of the object A, therefore, the endoscopic apparatus 100 is used to observe the object A with a certain space from the objective lens 112. When an enlarged image is required, so-called software zooming is performed rather than bringing the objective lens 112 close to the object A. In the software zooming, the input device 145 is used to input an instruction to enlarge an image so that the image processing unit 142 enlarges the frame image thereby to enlarge the image displayed on the display device 150. Even with the software zooming, image deterioration does not occur so much because the number of pixels is large.

According to the endoscopic apparatus 100 of the present embodiment, the LED element 125 with which large energy can be obtained is used as the light source of the illumination device 120. This allows bright illumination light and therefore a bright image to be obtained.

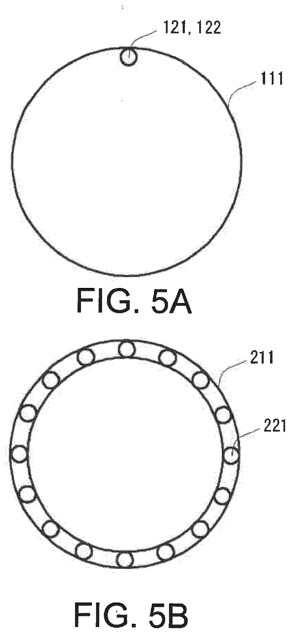

Moreover, the illumination light is guided by the optical fiber 121 disposed on the inner wall of the cylindrical part 111 and, therefore, the space in the hollow light guide part 113 of the cylindrical part 111 can be effectively used for guiding the light from the object A. This will be more specifically described. As illustrated in FIG. 5(b), a scheme of arranging optical fibers 221 on the circumference of a cylindrical part 211 is known as a form of the illumination of the endoscopic apparatus 100. According to this scheme, the space inside the cylindrical part 211 is occupied by the optical fibers 221 for illumination. This narrows the optical path for the image of an object and makes it difficult to project a large image on the imaging surface of the imaging element 131. In contrast, in the endoscopic apparatus 100, as illustrated in FIG. 5(a), the hollow light guide part 113 of the cylindrical part 111 can be widely utilized for guiding the light from the object A. In the case of the endoscopic apparatus 100, effective utilization of the hollow light guide part 113 is very advantageous because the outer diameter of the insertion unit 110 is limited.

In the present embodiment, the imaging device 130 and the control device 140 are separated and connected by cables. The handling is therefore easier than when a camera in which the imaging device 130 and the control device 140 are integrated is attached to the insertion unit 110. Moreover, the use environment is limited within an operation room; therefore, the length of the cables for connection can be 10 m or less and no serious problem will occur.

Furthermore, the bright illumination can prevent the captured image of the object A from being dark even when the image is enlarged using the software zooming.

### (Modified Examples)

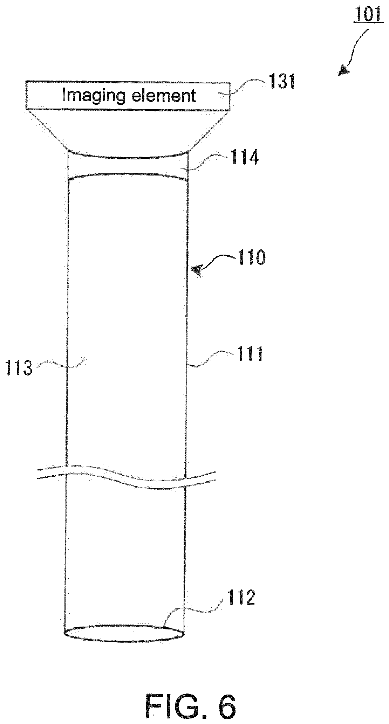

In the above embodiment, the light having passed through the cylindrical part 111 forms an image on the imaging element 131 without any beam transformation, but as illustrated in FIG. 6, a concave lens 114 may be disposed at the base end portion of the cylindrical part 111 to enlarge the diameter of the light flux having passed through the cylindrical part 111 so that the imaging element 131 is irradiated with the enlarged light flux.

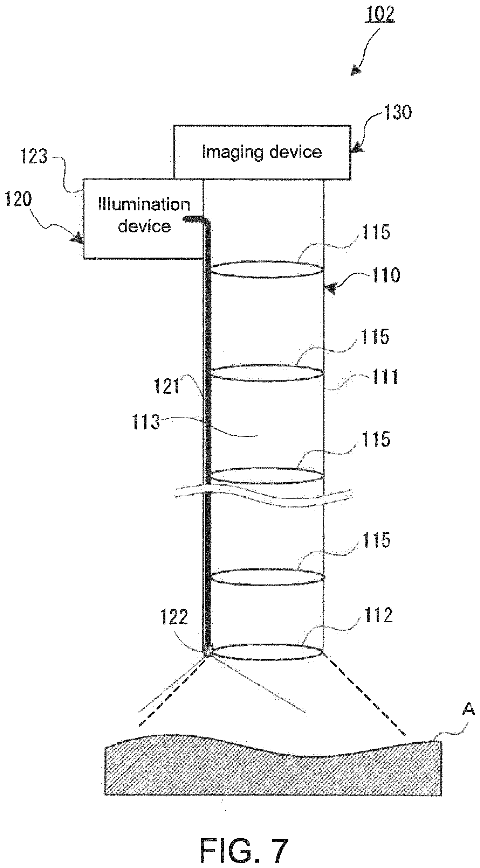

In FIG. 1, FIG. 2, FIG. 6, etc., each lens is illustrated as a single lens, but the form, size, and refractive index of the lens and the number of lens elements that constitute the lens may be freely designed, provided that the lens can converge the reflected light from the object A and form an image on the imaging element 131 of the imaging device 130. Moreover, the material of each lens may be freely selected from those, such as optical glass, plastic, and fluorite, which transmit light. In an embodiment, each lens may be combined with an additional lens such as a concave lens or an aspherical lens. In an embodiment, one lens (group) may be composed of a plurality of lenses. In an embodiment, as illustrated in FIG. 7, so-called relay lenses 115 may be arranged.

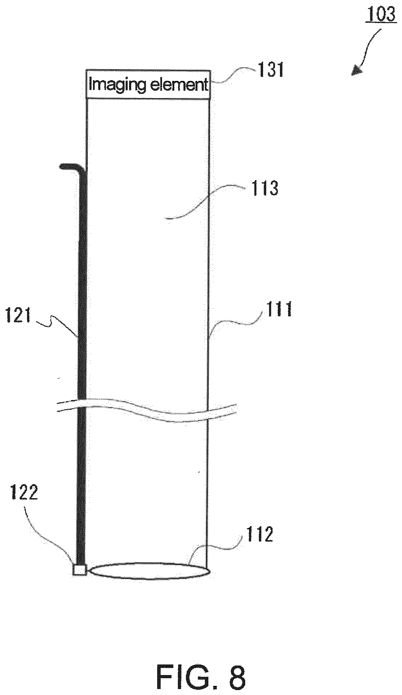

The optical fiber 121 disposed on the inner wall of the cylindrical part 111 is exemplified as a means for guiding the illumination light, but the optical fiber 121 may be disposed on the outer wall of the cylindrical part 111, as illustrated in FIG. 8.

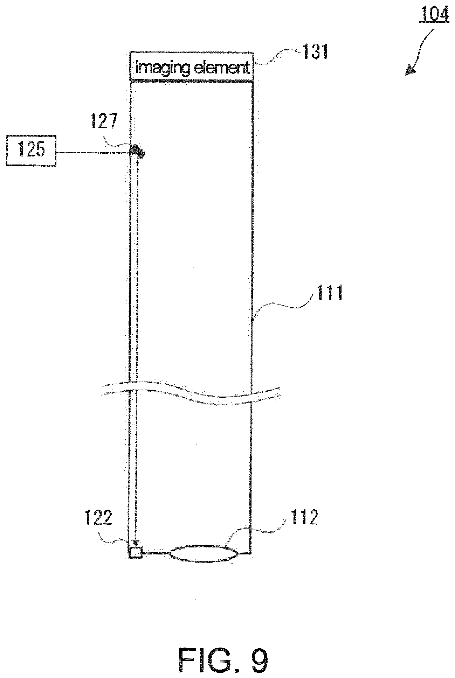

The present invention is not limited to an example in which light is guided by the optical fiber 121. In an alternative embodiment, as illustrated in FIG. 9, a reflecting mirror 127 may be used to guide the illumination light emitted from the LED element 125 to the diffusion layer 122.

In the above embodiment, the illumination light is white light that includes RGB components, so the reference wavelength λ is set as the wavelength of primary light of red having the longest wavelength among the three primary colors of light which constitute the illumination light. The present invention is not limited to this setting. For example, when the LED element 125 emits monochromatic light, its wavelength may be set as the reference wavelength λ and the pixel pitch P may be set larger than the reference wavelength λ. When the illumination light includes high-frequency but low-intensity light, such light cannot substantially contribute as the illumination light and is therefore not set as the reference wavelength λ. In addition or alternatively, when the illumination light includes light within a band in which the imaging element 131 is not sensitive, such light cannot substantially contribute as the illumination light and is therefore not set as the reference wavelength λ. In addition or alternatively, when the illumination light includes invisible light but the invisible light is within the sensitive band of the imaging element 131 and has high intensity to such an extent that the imaging is affected, it is preferred to set the wavelength of such light as the reference wavelength λ.

That is, the reference frequency (corresponding to λ) is the highest frequency of light included in the sensitive frequency band of the imaging element 131 among frequencies of light having intensity equal to or higher than a threshold that indicates a reference to such an extent that the imaging is affected.

The emission light of the LED element 125 is used for illumination without any conversion, but when the output light of the LED element 125 is used for illumination, for example, after converting the frequency with a component having a frequency conversion function, such as a fluorescent substance, the reference wavelength λ may be set as the wavelength of light having the longest wavelength among the primary light of the illumination light after the frequency conversion.

The imaging element 131 may comprise pixels equivalent to or larger than 8K. The diffusion layer 122 may not be provided.

### [Description of Reference Numerals]

- 100 to 104: Endoscopic apparatus
- 110: Insertion unit
- 111, 211: Cylindrical part
- 112: Objective lens
- 113: Hollow light guide region
- 114: Concave lens
- 115: Relay lenses
- 120: Illumination device
- 121, 221: Optical fiber
- 122: Diffusion layer
- 123: Light source unit
- 125: LED element
- 126: Driver circuit
- 127: Reflecting mirror
- 130: Imaging device
- 131: Imaging element
- 132: Driver circuit
- 133: A/D conversion unit
- 134: Transmission unit
- 140: Control device
- 141: Control unit
- 142: Image processing unit
- 143: Storage unit
- 144: Input/output IF
- 145: Input device
- 150: Display device

## Claims

1. An endoscopic apparatus comprising:
an insertion unit configured to be inserted into a body cavity and guide light from an object;
an illumination means attached to the insertion unit and illuminating the object; and
an imaging element comprising 8K-level or higher-level pixels arranged in a matrix form, the imaging element receiving light reflected from the object and guided through the insertion unit and outputting imaging signals of the object,
wherein the pixels of the imaging element have a pitch equal to or larger than a longest wavelength of illumination light emitted from the illumination means.

2. The endoscopic apparatus as recited in claim 1, wherein the illumination means comprises an LED element and a light guiding means that guides light output from the LED element.

3. The endoscopic apparatus as recited in claim 1 or 2, wherein the illumination light output from the illumination means includes light having a plurality of frequencies within a sensitive frequency band of the imaging element, and the pitch of the pixels is larger than a value corresponding to a highest frequency among the frequencies of light having intensity equal to or higher than a predetermined threshold.

4. The endoscopic apparatus as recited in claim 1, 2, or 3, wherein the imaging element is provided with a means that converts a pixel voltage to pixel data, and the endoscopic apparatus further comprises an image processing unit that creates frame data from the pixel data provided from the imaging element and processes the frame data, a display device that is connected to the image processing unit and displays the frame data, and a cable of 1 to 10 m that connects between the imaging element and the image processing unit.
